(19) Europäisches Patentamt
European Patent Office
Office européen des brevets

(11) EP 0 458 384 B1

(12) **FASCICULE DE BREVET EUROPEEN**

(45) Date de publication et mention
de la délivrance du brevet:
**28.02.1996 Bulletin 1996/09**

(51) Int. Cl.⁶: **A61B 8/06**

(21) Numéro de dépôt: **91201109.5**

(22) Date de dépôt: **08.05.1991**

(54) **Dispositif de mesure et de visualisation par échographie ultrasonore de débit d'un écoulement sanguin et de dilatation du vaisseau associé**

Mess- und Anzeigevorrichtung für die Geschwindigkeit der Blutströmung mittels Ultraschallechographie

Measuring and display device for the rate of blood flow using ultrasonic echography

(84) Etats contractants désignés:
**AT DE FR GB**

(30) Priorité: **22.05.1990 FR 9006361**

(43) Date de publication de la demande:
**27.11.1991 Bulletin 1991/48**

(73) Titulaires:
• **LABORATOIRES D'ELECTRONIQUE PHILIPS S.A.S.**
**F-94450 Limeil-Brévannes (FR)**
Etats contractants désignés:
**FR**
• **Philips Electronics N.V.**
**NL-5621 BA Eindhoven (NL)**
Etats contractants désignés:
**DE GB AT**

(72) Inventeur: **Bonnefous, Odile,**
**Société Civile S.P.I.D.**
**F-75008 Paris (FR)**

(74) Mandataire: **Pyronnet, Jacques et al**
**F-75008 Paris (FR)**

(56) Documents cités:
EP-A- 0 075 284          EP-A- 0 081 045
EP-A- 0 225 667          GB-A- 2 156 985
US-A- 3 631 849          US-A- 4 370 985

• **INTERNATIONAL CONFERENCE ON BIOMEDICAL TRANSDUCERS 03 novembre 1975, PARIS (FR) pages 271 - 275; J.P. ATHERTON: "MESURE DE LA PRESSION ET DU DEBIT DES LIQUIDES BIOLOGIQUES - mesure non -invasive de la tension arterielle humaine"**

## Description

La présente invention concerne un dispositif de mesure et de visualisation de paramètres physiologiques d'un écoulement sanguin dans un vaisseau comprenant une première unité de mesure pour échographie ultrasonore de la vitesse V(t,z) dudit écoulement sanguin en fonction du temps t et de la profondeur d'exploration z, la mesure de la vitesse V(t,z) étant indépendante de la fréquence de l'onde ultrasonore utilisée, et comprenant une mémoire de stockage des échantillons de vitesse V(t,z).

L'invention trouve une application particulièrement avantageuse dans le domaine général de l'exploration échographique d'écoulements sanguins dans les vaisseaux, et plus particulièrement, dans celui de la mesure et de la visualisation, aux fins de diagnostic, de paramètres physiologiques caractéristiques desdits écoulements.

L'étude clinique par échographie ultrasonore des écoulements sanguins est réalisée actuellement avec des appareils mettant en oeuvre l'effet Doppler qui rend compte de la différence de fréquence, appelée fréquence Doppler $f_D$, entre l'onde émise par un transducteur piézoélectrique et l'onde reçue après intéraction avec l'écoulement considéré. La vitesse V du flux sanguin est liée à $f_D$ par la relation :

$$f_D = 2(V/C)f_E \cos\theta \qquad (1)$$

$f_E$ étant la fréquence de l'onde ultrasonore émise, $\theta$ l'angle entre le faisceau ultrasonore et la direction de l'écoulement, et C la vitesse de propagation du son.

Les dispositifs Doppler connus permettent, notamment, d'accéder à des paramètres intéressant particulièrement les praticiens comme le spectogramme des vitesses donnant la distribution de la vitesse d'écoulement V en fonction du temps t. Selon cette technique déjà ancienne, on peut citer notamment le brevet EP-A 0 075 284. Toutefois, le résultat obtenu est entaché d'une certaine imprécision liée de façon inhérente au principe même de la méthode et qui provient du fait que la fréquence $f_E$ émise par le transducteur piézoélectrique présente une certaine distribution qui se répercute sur la vitesse V via la relation (1) rappelée ci-dessus.

Aussi, le problème technique à résoudre par l'objet de la présente invention est de réaliser un dispositif de mesure et de visualisation de paramètres physiologiques d'un écoulement sanguin, conforme au préambule, qui permettrait d'obtenir, outre un spectogramme des vitesses plus précis, la détermination de paramètres physiologiques supplémentaires avec la précision voulue, inaccessible jusqu'alors.

Parmi les dispositifs pouvant donner une mesure de vitesses indépendantes de la fréquence ultrasonore, on peut citer ceux qui fonctionnent sur le principe de la corrélation temporelle déjà décrite dans la demande de brevet européen n° 0 225 667, et dont l'unité de mesure de la vitesse d'écoulement comprend un circuit d'intercorrélation délivrant à partir de deux échos successifs des valeurs de fonction de corrélation, et un circuit de multiplexage-interpolation fournissant à partir desdites valeurs des fonctions de corrélation une estimée de la vitesse V(t,z).

La précision obtenue sur la mesure de la vitesse d'écoulement permet d'envisager, outre la visualisation du flux sanguin en mode M, par exemple, et la représentation du spectogramme des vitesses, de calculer d'autres paramètres physiologiques jusque là inaccessibles avec les systèmes Doppler conventionnels du fait de leur mauvaise résolution en profondeur.

Grâce à la précision ainsi obtenue les inconvénients de l'art antérieur sont atténués et supprimés et le dispositif de mesure et de visualisation défini en préambule est remarquable en ce qu'il comporte en outre en combinaison des premiers circuits de calcul du débit instantané Q(t) déduit desdits échantillons de vitesse V(t,z) ainsi qu'une deuxième unité de mesure de la vitesse radiale $V_1(t,z)$ et $V_2(t,z)$ de déplacement des deux parois de vaisseau qui bornent diamétralement ledit écoulement sanguin, des mémoires de stockage des valeurs de vitesse $V_1(t,z)$ et $V_2(t,z)$, des deuxièmes circuits de calcul comprenant un circuit de calcul de l'énergie locale $E'(t,z)$ du signal d'écho $S_n$ amplifié non débarrassé des échos fixes, qui calcule :

$$E'(t,z) = \sum_n S_n^2(t,z) ,$$

suivi d'un circuit de calcul d'épaisseurs respectives $d_1 = z_4 - z_3$ et $d_2 = z_6 - z_5$ desdites parois constitué par un détecteur de seuil de valeur $E'_0$ ajustable pour la détermination des valeurs $z_3$, $z_4$, $z_5$, $z_6$, deux circuits de calcul des vitesses moyennes respectives $\hat{V}_1(t_0)$ et $\hat{V}_2(t_0)$ desdites parois pour chaque valeur de temps $t_0$ constitués par un additionneur donnant $\Sigma_{d1} V_1(t,z)$ (respectivement $\Sigma_{d2} V_2(t,z)$) et par un diviseur par $M_1$ (respectivement $M_2$), $M_1$ et $M_2$ étant le nombre d'échantillons de mesure sur le segment $[z_3, z_4]$ (respectivement $[z_5, z_6]$), un circuit de calcul du déplacement de chaque paroi constitué par un additionneur donnant :

$$D_1(t) = \Sigma_t \hat{V}_1(t_0) \text{ et } D_2(t) = \Sigma_t \hat{V}_2(t_0) ,$$

et un circuit de calcul du déplacement symétrique des parois constitué par un soustracteur et un diviseur par 2 donnant sous forme d'échantillons temporels la variation de rayon instantanée dudit vaisseau $\Delta r(t) = (D_2(t)-D_1(t))/2$ et des moyens de visualisation des courbes Q(t) et $\Delta r(t)$ en fonction du temps t.

Parmi les paramètres dont la présente invention autorise la détermination avec une bonne précision figurent la vitesse moyenne $\hat{V}(t)$ et le débit instantané Q(t) du flux sanguin dans un vaisseau et aussi les vitesses moyennes respectives $\hat{V}_1(t_0)$ et $\hat{V}_2(t_0)$ des parois du vaisseau, ces deux dernières permettant, après intégration par rapport au temps, d'obtenir la loi de déplacement de chaque paroi, $D_1(t)$ et $D_2(t)$. De ces déplacements, on déduit la variation de rayon instantanée du vaisseau $\Delta r(t)$ et il est intéressant de noter que la fonction $\Delta r(t)$ est en relation linéaire avec la variation de pression instantanée du sang dans le vaisseau, $\Delta p(t)$. La connaissance simultanée du débit et de la pression permet d'effectuer divers calculs et se prête à plusieurs représentations fort utiles pour le praticien.

Un mode de réalisation préféré du dispositif de mesure et de visualisation selon l'invention est remarquable en ce que lesdits premiers circuits de calcul du débit instantané $Q(t)$ comprennent un circuit de calcul de l'énergie locale débarrassée des échos fixes $E(t,z)$, suivi d'un circuit de calcul du diamètre d'écoulement $D(t) = z_2(t)-z_1(t)$ constitué par un détecteur de seuil de valeur $E_0$, $z_2(t)$ et $z_1(t)$ étant définis par $E(t,z_1(t)) = E(t,z_2(t)) = E_0$, un circuit de calcul de la vitesse moyenne $\hat{V}(t)$ de l'écoulement constitué par un additionneur donnant $\Sigma_D V(t,z)$ et par un diviseur par M, M étant le nombre d'échantillons de mesure sur le segment $[z_1, z_2]$, et un circuit de calcul du débit $Q(t)$ comprenant, d'une part un circuit de calcul du centre de gravité $z_0(t)$ de l'écoulement, constitué d'un additionneur donnant $\Sigma_D V(t,z)z$ et d'un diviseur par la vitesse moyenne $\hat{V}(t)$ et d'autre part un additionneur donnant $\Sigma_D V(t,z)|z-z_0|$ suivi d'un multiplieur par une constante A fonction de l'angle $\theta$ de l'écoulement avec le faisceau ultrasonore.

Un mode de représentation particulièrement intéressant du débit et de la variation de pression combinés consiste à calculer et à visualiser la courbe du cycle cardiaque, paramétrée en temps, constituée par les points obtenus en portant en ordonnées les valeurs-échantillons de la fonction $\Delta r(t)$ et en abscisses les valeurs-échantillons de la fonction dudit débit instantané $Q(t)$.

De là on peut déduire notamment, à partir de la mesure de la surface du cycle et de la surface sous-tendue par le cycle, le calcul de l'efficacité cardiaque pour la tranche d'épaisseur axiale dx du vaisseau analysé.

Un autre mode de réalisation préféré du dispositif de mesure et de visualisation selon l'invention compatible avec les précédents est remarquable en ce qu'il comporte en outre un dispositif de détection des abscisses d'un minimum, à l'instant $t_1$, d'un maximum suivant, à l'instant $t_3$, et d'un deuxième minimum suivant, à l'instant $t_2$, de la fonction $\Delta r(t)$, la durée $\Delta t = t_2-t_1$ étant celle d'un cycle cardiaque, un processeur de décomposition en série de Fourier qui reçoit les signaux échantillonnés dans le temps $\Delta r(t)$ et $Q(t)$ entre les instants $t_1$ et $t_2$ et en déduit des échantillons fréquentiels $\Delta r(kf_0)$ et $Q(kf_0)$, $f_0 = 1/\Delta t$ étant la fréquence fondamentale du cycle cardiaque et k le rang de l'harmonique considéré, et un processeur de calcul d'impédance qui fournit, pour chaque harmonique l'amplitude $\|\Delta r_k/Q_k\|$ et la phase $Arg(\Delta r_k/Q_k)$ de l'impédance de la section de vaisseau analysée.

Des moyens de visualisation de l'amplitude et de la phase de l'impédance point par point, pour chaque harmonique de cycle cardiaque permettent de bien caractériser cette impédance.

On notera que les mesures et visualisations de paramètres physiologiques d'un écoulement sanguin dans un vaisseau indiquées ci-dessus sont obtenues sans l'aide d'un ou de plusieurs cathéters (ou sondes invasives), soit sans traumatisme pour le patient ni risques d'obtenir des mesures faussées par l'appareil d'observation même.

La description qui suit en regard des dessins annexés le tout donné à titre d'exemple fera bien comprendre comment l'invention peut être réalisée.

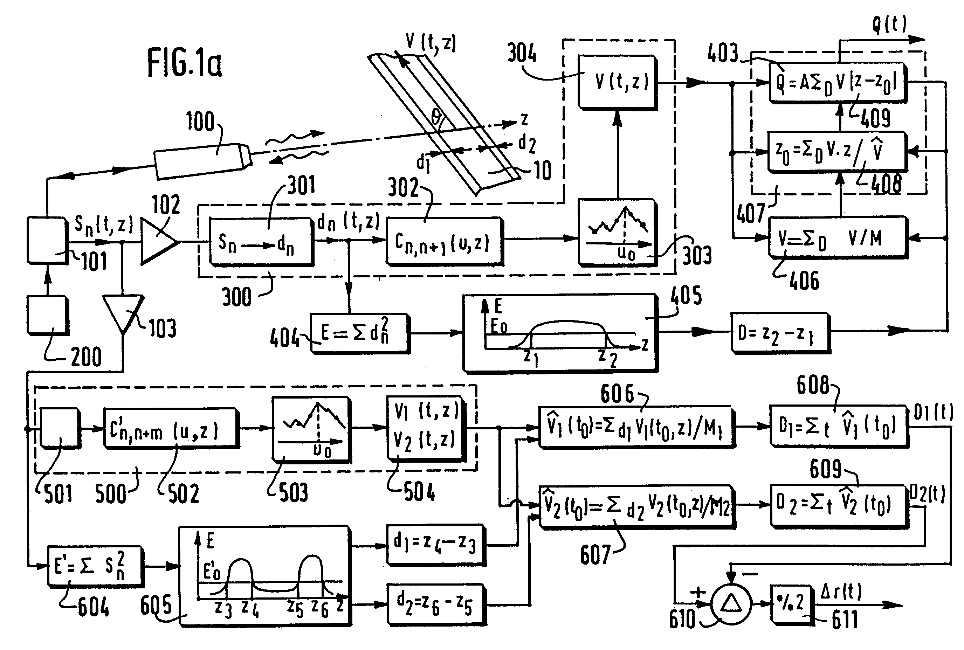

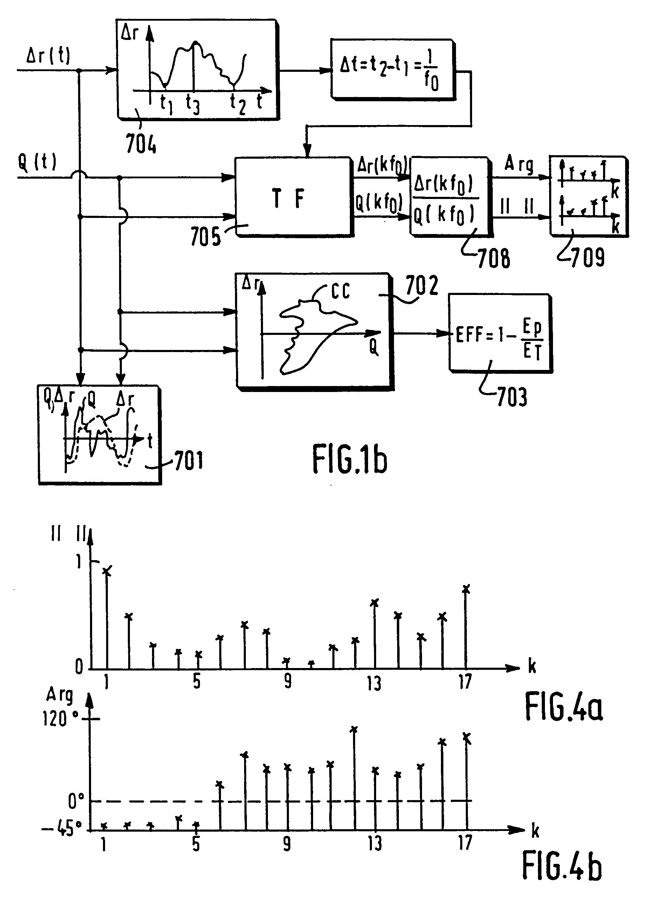

La réunion des figures 1a et 1b constitue le schéma synoptique d'un dispositif de mesure et de visualisation selon l'invention.

La figure 2 représente les fonctions débit $Q(t)$ et variation de rayon $\Delta r(t)$ qui caractérisent l'écoulement sanguin, en fonction du temps.

La figure 3 représente un cycle cardiaque tel qu'il est représenté à l'endroit du vaisseau analysé.

Les figures 4a et 4b représentent, pour les premiers harmoniques du cycle cardiaque, respectivement l'amplitude et la phase de l'impédance à l'endroit du vaisseau analysé.

Le schéma de la figure 1a montre un dispositif de mesure de paramètres physiologiques d'un écoulement sanguin 10. Ce dispositif comprend un transducteur piézoélectrique 100 qui peut être, par exemple, une barrette multiéléments. Un étage 200 d'émission relié au transducteur 100 assure la formation d'un faisceau ultrasonore d'exploration tandis qu'une première unité 300 de mesure traite les signaux échographiques renvoyés vers le transducteur 100 de façon à fournir une estimée de la vitesse $V(t,z)$ de l'écoulement en fonction du temps t et de la profondeur z d'exploration.

De façon classique, l'étage d'émission 200 comprend un séquenceur composé d'un oscillateur et d'un diviseur de fréquence qui commande à la fréquence de récurrence 1/T choisie un générateur dont les signaux électriques d'excitation sont envoyés vers le transducteur 100 qui les convertit en trains périodiques de signaux impulsionnels ultrasonores. Un séparateur 101 des étages d'émission 200 et de mesure 300 est inséré entre le transducteur 100 et lesdits étages 200, 300 et évite l'aveuglement des circuits de mesure par les signaux d'émission.

L'unité 300 de mesure de la vitesse $V(t,z)$ montrée à la figure 1 comporte un éliminateur 301 d'échos fixes, à deux points par exemple, qui fournit du signal reçu $S_n(t,z)$ après amplification par un amplificateur 102 un signal $d_n(t,z)$ débarrassé de ses composantes fixes dues aux réflexions spéculaires sur les parois du vaisseau sanguin examiné. Le signal $d_n(t,z)$ issu de l'éliminateur 301 d'échos fixes est ensuite traité selon la méthode dite d'intercorrélation décrite dans la demande de brevet européen n° 0 225 667 et qui utilise le fait que les signaux ultrasonores rétrodiffusés par une cible en mouvement sont reliés par l'équation suivante :

$$d_{n+1}(t) = d_n(t-\tau)$$

ceci signifie que le signal n+1 est la réplique du signal n précédent à un décalage temporel $\tau$ près. Ce dernier

représente le temps supplémentaire nécessaire à l'onde ultrasonore pour parcourir le trajet transducteur-cible-transducteur, d'un tir à l'autre. Autrement dit :

$$\tau = 2VT/C$$

où V est la vitesse de la cible et C la vitesse du son. On voit qu'une mesure de $\tau$ permet de mesurer la vitesse V cherchée.

La fonction d'intercorrélation entre $d_n(t)$ et $d_{n+1}(t)$ définie par :

$$C_{n,n+1}(to,u) = \int_{to}^{to+W} d_{n+1}(t+u)d_n(t)dt$$

vérifie :

$$C_{n,n+1}(to,u)=C_{n,n}(to,u-\tau)$$

Le temps to est lié à la profondeur d'exploration z par to=2z/C , et W est la largeur de la fenêtre d'intégration.

La fonction $C_{nn}(to,u)$ est une fonction d'autocorrélation, et, de ce fait, est maximale pour u=o . Ainsi, une mesure du décalage temporel $\tau$, et donc de la vitesse V, peut se faire en cherchant pour quel paramètre u la fonction $C_{n,n+1}(to,u)$ est maximale. A cet effet, la fonction d'intercorrélation est échantillonné, à un pas d'échantillonnage $\Delta t$, entre $u_{min}=-I\Delta t$ et $u_{max}=I\Delta t$ par pas de 1 de façon à obtenir 2I+1 valeurs de fonctions de corrélation. La valeur maximum de ces 2I+1 valeurs correspondant à u=uo permet de mesurer $\tau$ en utilisant l'égalité $\tau$=uo . Le calcul des fonctions de corrélation est réalisé par le circuit 302 d'intercorrélation montré à la figure 1.

Afin de s'affranchir des erreurs inhérentes à l'échantillonnage dans la détermination du maximum de la fonction de corrélation, il est possible d'utiliser un circuit 303 de multiplexage-interpolation fournissant à partir des valeurs des fonctions de corrélation une estimée plus précise de la vitesse et la valeur du pic de corrélation correspondant. La demande de brevet européen n°0 225 667 donne un exemple de ce type de traitement du signal échographique dans lequel la corrélation entre signaux est une corrélation dite "1 bit" en ce sens que les signaux $d_{n+1}$ et $d_n$ utilisés précédemment sont réduits au signe du signal ultrasonore. On sait que dans ce cas, le pic de la fonction de corrélation est de forme triangulaire isocèle. La connaissance de cette forme permet à partir du point le plus haut et de ses deux voisins de reconstituer complètement, par interpolation linéaire, le pic de corrélation et donc de déterminer avec précision l'emplacement de uo.

Contrairement aux vélocimètres Doppler traditionnels, la vitesse d'écoulement V(t,z) ainsi déterminée présente l'avantage d'être insensible à la dispersion de la fréquence de l'onde ultrasonore utilisée, ce qui permet d'envisager une exploitation beaucoup plus complète des résultats.

Les valeurs trouvées pour la vitesse V(t,z) sont stockées dans une mémoire 304 de stockage en vue de traitements ultérieurs.

La figure 1a montre comment peuvent être calculés certains paramètres physiologiques caractéristiques de l'écoulement. Il s'agit d'abord du diamètre D(t) de l'écoulement dont la mesure met en oeuvre, en sortie de l'éliminateur 301 d'échos fixes, un circuit 404 de calcul de l'énergie locale :

$$E(t,z) = \sum_n d_n^2(t,z)$$

et un circuit 405 de calcul du diamètre d'écoulement $D(t) = z_2(t)-z_1(t)$ constitué par un détecteur de seuil de valeur Eo ajustable, $z_1(t)$ et $z_2(t)$ étant définis par $E(t,z_1(t)) = E(t,z_2(t)) = Eo$ .

Connaissant, à chaque instant t, le diamètre D(t) de l'écoulement, il est possible de calculer la vitesse moyenne $\hat{V}(t)$ du flux sanguin grâce à un circuit 406 constitué par un additionneur donnant $\Sigma_D V(t,z)$ et par un diviseur par M, M étant le nombre d'échantillons de la vitesse V(t,z) utilisés sur le segment $[z_1,z_2]$.

De même, le débit instantané Q(t) peut être mesuré par un circuit 407 comprenant, d'une part, un circuit 408 de calcul du centre de gravité $z_o(t) = \Sigma_D V(t,z)z/\hat{V}(t)$ , constitué d'un additionneur donnant $\Sigma_D V(t,z)z$ et d'un diviseur par la vitesse moyenne $\hat{V}(t)$ calculée par le circuit 406, et, d'autre part, un additionneur 401 donnant $\Sigma_D V(t,z)|z-z_o|$ suivi d'un multiplieur par la constante $A = \pi cos\theta/sin^2\theta$, $\theta$ étant l'angle de l'écoulement avec le faisceau ultrasonore.

Un autre paramètre physiologique à mesurer avec précision et qui est nécessaire à la mise en oeuvre de l'invention est la variation de pression sanguine instantanée, $\Delta p(t)$ à l'intérieur du vaisseau analysé. De façon connue, en vertu du comportement élastique des parois du vaisseau, la fonction $\Delta p(t)$ est reliée à la variation de rayon instantanée du vaisseau, $\Delta r(t)$ par la relation : $\Delta p(t) = K \Delta r(t)$ , K étant une constante pour la partie de vaisseau analysée. Cette constante dépend à la fois des caractéristiques mécaniques (élastiques) du vaisseau et de l'angle $\theta$. Pour la mesure de $\Delta r(t)$, il est prévu une deuxième unité de mesure 500 de la vitesse radiale $V_1(t,z)$ et $V_2(t,z)$ de déplacement des deux parois de vaisseau qui bornent diamétralement l'écoulement sanguin ainsi que d'autres circuits de mémorisation et de calcul comme représenté au bas de la figure 1a.

Le signal $S_n(t,z)$ en sortie du séparateur 101 est fourni, par l'intermédiaire d'un amplificateur 103 à l'unité de mesure 500. L'amplification par l'amplificateur 103 est moins grande que celle de l'amplificateur 102 de façon à adapter la dynamique des signaux d'écho sur les parois du vaisseau aux étages de calcul disposés en aval. Cette particularité est associée au fait que la deuxième unité de mesure ne comporte pas d'éliminateur d'échos fixes étant donné que ce sont précisément les signaux d'écho de paroi que l'on traite à présent. Dans l'unité de mesure 500, le signal $S_n(t,z)$ issu de l'amplificateur 103 est d'abord stocké dans une mémoire 501 puis traité selon la méthode d'intercorrélation décrite dans la demande de brevet européen n° 0 225 667, de façon semblable à celle décrite ci-dessus pour l'unité 300. Cependant, la période de récurrence de traitement peut avantageusement être choisie comme un multiple m de la période de récurrence d'émission T. En effet, les vitesses de déplacement des parois étant très faibles, typiquement égales à 0,5 cm/s, il est préférable de corréler des signaux successifs plus espacés (de m tirs) dans le temps et donc non adjacents. L'opération de corrélation correspondante, au bloc 502, peut être notée sous la forme : $C_n$, $_{n+m}$ $(u,z)$. Afin de s'affranchir des erreurs inhérentes à l'échantillonnage dans la détermination du maximum de la fonction de corrélation, on peut utiliser un circuit 503 de multiplexage-interpolation. Les valeurs trouvées pour les vitesses $V_1(t,z)$ et $V_2(t,z)$ sont stockées dans une mémoire 504 en vue de traitements ultérieurs.

La figure 1a montre comment peuvent être calculés d'autres paramètres caractéristiques du vaisseau, aboutissant au calcul de la fonction $\Delta r(t)$. Il s'agit d'abord de l'épaisseur des parois $d_1(t)$ et $d_2(t)$ diamétralement opposées dont la mesure met en oeuvre, en sortie de l'amplificateur 103 un circuit 604 de calcul de l'énergie locale

$$E'(t,z) = \sum_n S_n^2(t,z)$$

et un circuit 605 de calcul des épaisseurs de parois $d_1(t) = z_4(t)-z_3(t)$ et $d_2(t) = z_6(t)-z_5(t)$ constitué par un détecteur de seuil de valeur $E'_0$ ajustable. Pour éviter toute ambiguïté sur l'emplacement des parois, on peut prévoir d'afficher sur un écran, non représenté une image du vaisseau étudié, selon la représentation dite en "mode M", de l'évolution au cours du temps du profil de vitesse des parois du vaisseau et prévoir que l'utilisateur, muni d'un stylo optique (souris) localise sur l'écran un point à l'intérieur de chaque paroi, point à partir duquel peuvent alors s'effectuer les sommations (intégrations) qui suivent, en direction des bornes $z_3$ et $z_4$, respectivement $z_5$ et $z_6$. Le codage de la vitesse en mode M est celui utilisé dans les systèmes CFM (Color Flow Mapping) : par exemple le rouge code un sens de déplacement, le bleu le sens inverse et l'intensité de la vitesse est codée par l'intensité de la couleur.

Connaissant, à chaque instant $t_0$, les épaisseurs $d_1(t)$ et $d_2(t)$ des parois, il est possible de calculer les vitesses moyennes $\hat{V}_1(t_0)$ et $\hat{V}_2(t_0)$ des parois grâce à des circuits 606 et 607 constitués chacun par un additionneur et par un diviseur donnant respectivement :

$$\hat{V}_1(t_0) = \Sigma_{d1}\, V_1(t_0,z)/M_1$$

$$\hat{V}_2(t_0) = \Sigma_{d2}\, V_2(t_0,z)/M_2$$

$M_1$ et $M_2$ étant le nombre d'échantillons de mesure sur le segment $[z_3\text{-}z_4]$, respectivement $[z_5\text{-}z_6]$.

Une deuxième intégration, (sommation d'échantillons) dans le temps de la vitesse moyenne, permet d'obtenir avec précision la fonction déplacement de chaque paroi, ce qui est réalisé par les additionneurs 608 et 609 :

$$D_1(t) = \sum_{t_0=0}^{t_0=t} \hat{V}_1(t_0)$$

$$D_2(t) = \sum_{t_0=0}^{t_0=t} \hat{V}_2(t_0)$$

On notera que les vitesses des parois, perpendiculaires à celles de l'écoulement sanguin, sont très faibles, de l'ordre de 0,5 cm/s. D'autre part on ne souhaite extraire que les mouvements de sens contraires des parois, c'est-à-dire les mouvements symétriques par rapport au milieu du vaisseau. Il faut donc éliminer les mouvements allant dans le même sens, c'est-à-dire les mouvements antisymétriques dûs soit à un déséquilibre des forces de pression de part et d'autre du vaisseau, soit au léger mouvement du transducteur. Le déplacement symétrique moyen s'obtient par soustraction $D_2(t)$-$D_1(t)$ dans un soustracteur 610 puis division par 2 au moyen d'un diviseur 611 qui fournit la variation de rayon instantanée $\Delta r(t)$ :

$$\Delta r(t) = 1/2\ (D_2(t) - D_1(t)).$$

La figure 1b qui constitue la suite du schéma de la figure 1a montre l'exploitation que l'on peut faire de la fourniture simultanée des paramètres physiologiques $Q(t)$ et $\Delta r(t)$, à l'usage du praticien. Cette exploitation consiste en l'affichage qui est, de préférence, effectué en même temps sur un écran unique (non représenté) constituant des moyens de visualisation.

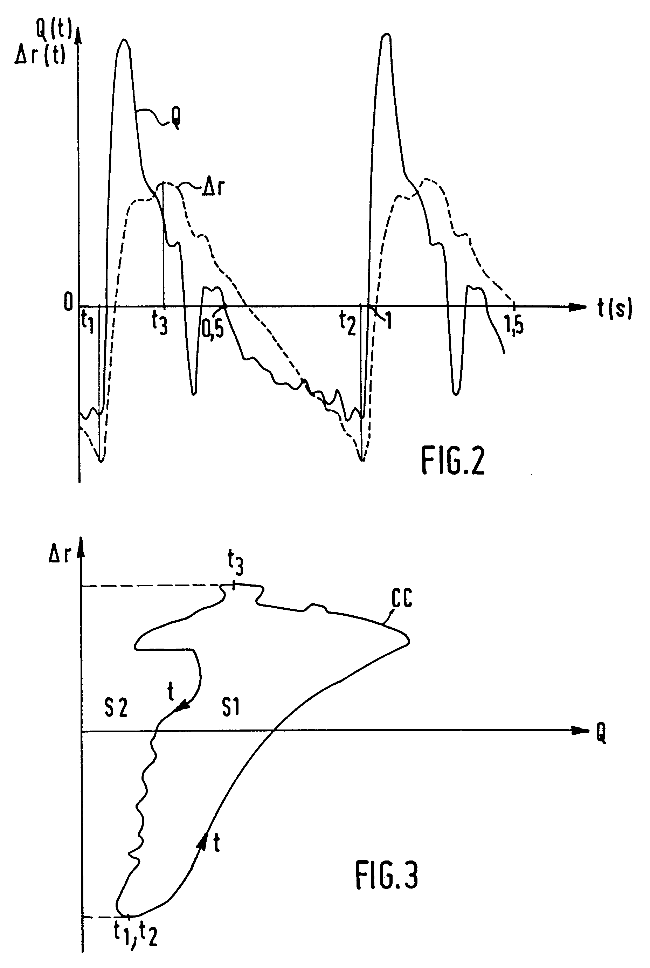

En premier lieu, les courbes Q et $\Delta r$ sont tracées en fonction du temps t selon les mêmes axes de coordonnées au bloc 701 (figure 1b) et à la figure 2, le débit Q étant représenté en trait plein et la variation de rayon $\Delta r$ en trait interrompu. Les courbes obtenues sont naturellement périodiques, au rythme du cycle cardiaque. Ce cycle peut être repéré par rapport aux abscisses des minima et maxima de la fonction $\Delta r(t)$ par exemple. Sur la figure 2 où les courbes $\Delta r$ et Q son débarrassées de leur composante continue pour faciliter leur comparaison, on observe deux minima successifs, aux temps $t_1$ et $t_2$ et un maximum intercalé entre ces deux minima, au temps $t_3$.

Au bloc 702, figure 1b et à la figure 3 est visualisée la courbe CC du cycle cardiaque, paramétrée en temps, constituée par les points obtenus en portant en ordonnées les valeurs de la fonction $\Delta r(t)$ et en abscisses les valeurs de la fonction du débit instantané $Q(t)$, la courbe Q étant représentée avec les valeurs effectivement mesurées. On a repéré sur la courbe CC les minima, confondus, obtenus aux instants $t_1$ et $t_2$ et le maximum obtenu à l'instant $t_3$. Alors que la valeur du débit est ici une mesure de la valeur réelle de l'écoulement sanguin dans le vaisseau analysé, on notera que la fonction $\Delta r(t)$ concernant le déplacement des parois est en relation linéaire avec la variation de pression instantanée, c'est-à-dire définie à une constante multiplicative près conformément à la relation précitée : $\Delta p = K\Delta r$ . Ceci n'est pas gênant pour le praticien en l'occurrence, étant donné que la forme du cycle cardiaque peut, par elle-même, donner au praticien des renseignements utiles sur d'éventuelles anomalies que peut présenter ce cycle. Ceci ne nuit pas non plus à une détermination de l'efficacité du cycle. On remarque en effet que la surface fermée par la courbe CC sur tout le cycle (surface $S_1$) est proportionnelle aux pertes par viscosité, $E_p$, pour la tranche de vaisseau analysée, alors que l'énergie totale $E_T$ (énergie cinétique du sang) est donnée selon la même proportionnalité par l'intégrale de cette courbe entre les instants $t_1$ et $t_3$, soit la surface $S_1 + S_2$, $S_2$ étant la surface adjacente sous-tendue par le cycle en direction de l'axe des ordonnées. L'efficacité du vaisseau (de l'artère) pendant la durée du cycle cardiaque, EFF, peut alors être définie par :

$$EFF = \frac{E_T - E_P}{E_T} = 1 - \frac{E_P}{E_T}$$

et affichée sur l'écran, au bloc 703.

Sur un plan mathématique, les intégrales à calculer s'expriment à un coefficient multiplicatif près qui n'intervient pas dans le calcul de EFF, par :

$$\int_{t_1}^{t_2} Q(t)\, d\Delta r(t) \text{ pour } E_p \text{ et :}$$

$$\int_{t_1}^{t_3} Q(t)\, d\Delta r(t) \text{ pour } E_T.$$

L'efficacité EFF peut encore être définie comme le rapport entre l'énergie cinétique restante et l'énergie totale fournie pour la portion de vaisseau analysée, pendant un cycle et dépend donc de la rugosité et/ou de l'étranglement locaux de la paroi du vaisseau.

La partie haute du schéma de la figure 1b et les figures 4a et 4b illustrent le calcul d'impédance transposé au flux sanguin et qui peut être déduit des fonctions périodiques $Q(t)$ et $\Delta r(t)$. Cette impédance peut être définie comme le rapport de la pression au débit $p(t)/Q(t)$. Ici encore la pression et donc l'impédance n'est obtenue qu'à une constante multiplicative près. Il est prévu un circuit de détection de deux minima successifs de la fonction $\Delta r(t)$, circuit 704 qui fournit la durée du cycle $t_2 - t_1 = \Delta t = 1/f_0$ , $f_0$ étant la fréquence fondamentale du cycle. La donnée $f_0$ est fournie à un processeur de décomposition en série de Fourier TF, 705, qui reçoit par ailleurs les signaux échantillonnés dans le temps $\Delta r(t)$ et $Q(t)$ et, par transformation temporelle-fréquentielle, les convertit en des échantillons fréquentiels à valeurs complexes $\Delta r(kf_0)$ et $Q(kf_0)$, k étant le rang de l'harmonique considéré par rapport au fondamental de fréquence $f_0$. Ces échantillons fréquentiels sont ensuite fournis à un processeur de calcul d'impédance 708 qui fournit, par division de nombres complexes, pour chaque harmonique, l'amplitude normée $\|\Delta r_k/Q_k\|$ et la phase $\mathrm{Arg}(\Delta r_k/Q_k)$ de l'impédance de la section de vaisseau analysée. Ces amplitudes normées et ces phases sont affichées par des moyens de visualisation 709, représentés plus en détail aux figures 4a et 4b où l'on a indiqué en abscisses le rang k des harmoniques jusqu'à l'harmonique 17.

On notera qu'au bloc 702, figure 1b, il est possible d'illustrer en séquence de façon non représentée, le cycle cardiaque pour chaque harmonique considéré, chaque courbe ainsi obtenue, paramétrée en temps comme la courbe CC, revêtant la forme d'une ellipse. On peut ainsi déduire la contribution énergétique due à chaque harmonique du cycle cardiaque.

**Revendications**

1. Dispositif de mesure et de visualisation de paramètres physiologiques d'un écoulement sanguin dans un vaisseau comprenant une première unité de mesure (300) pour échographie ultrasonore de la vitesse $V(t,z)$ dudit écoulement sanguin en fonction du temps t et de la profondeur d'exploration z, la mesure de la vitesse $V(t,z)$ étant indépendante de la fréquence de l'onde ultrasonore utilisée, et comprenant une mémoire (304) de stockage des échantillons de vitesse $V(t,z)$, caractérisé en ce qu'il comporte en outre des premiers circuits de calcul (403 à 409) du débit instantané $Q(t)$ déduit desdits échantillons de vitesse $V(t,z)$ ainsi qu'une deuxième unité de mesure (500) de la vitesse radiale $V_1(t,z)$ et $V_2(t,z)$ de déplacement des deux parois de vaisseau qui bornent diamétralement ledit écoulement sanguin, des mémoires (504) de stockage des valeurs de vitesse $V_1(t,z)$ et $V_2(t,z)$, des deuxièmes circuits de calcul comprenant un circuit de calcul (604) de l'énergie locale $E'(t,z)$ du signal d'écho $S_n$ amplifié non débarrassé des échos fixes, qui calcule :

$$E'(t,z) = \sum_n S_n^2(t,z) \ ,$$

suivi d'un circuit de calcul (605) d'épaisseurs respectives $d_1 = z_4 - z_3$ et $d_2 = z_6 - z_5$ desdites parois constitué par un détecteur de seuil $E'_0$ ajustable pour la détermination des valeurs $z_3$, $z_4$, $z_5$, $z_6$, deux circuits de calcul (606, 607) des vitesses moyennes respectives $\hat{V}_1(t_0)$ et $\hat{V}_2(t_0)$ desdites parois pour chaque valeur de temps $t_0$ constitués par un additionneur donnant $\Sigma_{d1}\, V_1(t,z)$ respectivement $\Sigma_{d2}\, V_2(t,z)$ et par un diviseur $M_1$ respectivement $M_2$, $M_1$ et $M_2$ étant le nombre d'échantillons de mesure sur le segment $[z_3, z_4]$ respectivement $[z_5,

$z_6$], un circuit de calcul (608, 609) du déplacement de chaque paroi constitué par un additionneur donnant :

$$D_1(t) = \Sigma_t \hat{V}_1(t_0) \text{ et } D_2(t) = \Sigma_t \hat{V}_2(t_0),$$

et un circuit de calcul du déplacement symétrique des parois constitué par un soustracteur (610) et un diviseur par 2 (611) donnant sous forme d'échantillons temporels la variation de rayon instantanée dudit vaisseau $\Delta r(t) = (D_2(t)-D_1(t))/2$ et des moyens de visualisation (701, 702) des courbes Q(t) et $\Delta r(t)$ en fonction du temps t.

2. Dispositif de mesure et de visualisation selon la revendication 1, caractérisé en ce que lesdits premiers circuits de calcul du débit instantané Q(t) comprennent un circuit de calcul (405) de l'énergie locale débarrassée des échos fixes E(t,z), suivi d'un circuit de calcul du diamètre d'écoulement $D(t) = z_2(t)-z_1(t)$ constitué par un détecteur de seuil de valeur $E_0$, $z_2(t)$ et $z_1(t)$ étant définis par :

$$E(t,z_1(t)) = E(t,z_2(t)) = E_0,$$

un circuit de calcul (406) de la vitesse moyenne $\hat{V}(t)$ de l'écoulement constitué par un additionneur donnant :

$$\Sigma_D V(t,z)$$

et par un diviseur M, M étant le nombre d'échantillons de mesure sur le segment $[z_1, z_2]$, et un circuit (407) de calcul du débit Q(t) comprenant, d'une part un circuit de calcul (408) du centre de gravité $z_0(t)$ de l'écoulement, constitué d'un additionneur donnant $\Delta_D V(t,z)z$ et d'un diviseur par la vitesse moyenne $\hat{V}(t)$ et d'autre part un additionneur (409) donnant :

$$\Delta_D V(t,z)|Z-Z_0|$$

suivi d'un multiplieur par une constante A fonction de l'angle $\theta$ de l'écoulement avec le faisceau ultrasonore.

3. Dispositif de mesure et de visualisation selon la revendication 1 ou 2, caractérisé en ce qu'il comporte en outre des moyens de visualisation (702) de la courbe du cycle cardiaque CC, paramétrée en temps, constituée par les points obtenus en portant en ordonnées les valeurs de la fonction $\Delta r(t)$ en relation linéaire avec la variation de pression instantanée dans le vaisseau et en abscisses les valeurs de la fonction dudit débit instantané Q(t).

4. Dispositif de mesure et de visualisation selon la revendication 3, caractérisé en ce qu'il comporte en outre un circuit (703) de calcul de l'efficacité cardiaque EFF définie par :

$$EFF = 1 - E_p/E_T,$$

relation dans laquelle $E_p$ est égale à la surface $S_1$ dudit cycle cardiaque et $E_T$ est égale à la surface du cycle augmentée de la surface adjacente $S_2$ sous tendue par le cycle en direction de l'axe des ordonnées.

5. Dispositif de mesure et de visualisation selon l'une des revendications 1 à 4, caractérisé en ce qu'il comporte en outre un dispositif (704) de détection des abscisses d'un minimum, à l'instant $t_1$, d'un maximum suivant, à l'instant $t_3$, et d'un deuxième minimum suivant, à l'instant $t_2$, de la fonction $\Delta r(t)$, la durée $\Delta t = t_2-t_1$ étant celle d'un cycle cardiaque, un processeur de décomposition en série de Fourier (705) qui reçoit les signaux échantillonnés dans le temps $\Delta r(t)$ et Q(t) entre les instants $t_1$ et $t_2$ et en déduit des échantillons fréquentiels $\Delta r(kf_0)$ et $Q(kf_0)$, $f_0 = 1/\Delta t$ étant la fréquence fondamentale du cycle cardiaque et k le rang de l'harmonique considéré, et un processeur (708) de calcul d'impédance qui fournit, pour chaque harmonique l'amplitude $|\Delta r_k/Q_k|$ et la phase $Arg(\Delta r_k/Q_k)$ de l'impédance de la section de vaisseau analysée.

6. Dispositif de mesure et de visualisation selon la revendication 5, caractérisé en ce qu'il comporte en outre des moyens de visualisation (709) de l'amplitude et de la phase de l'impédance point par point, pour chaque harmonique du cycle cardiaque.

7. Dispositif de mesure et de visualisation selon l'une quelconque des revendications 1 à 6, caractérisé en ce que la première unité de mesure de vitesse d'écoulement et la deuxième unité de mesure de vitesse de déplacement des parois comprennent chacune un circuit d'intercorrélation (302, 502) délivrant, à partir de deux échos successifs, des valeurs de fonctions de corrélation, et un circuit de multiplexage-interpolation (303, 503) fournissant à partir desdites valeurs de corrélation une estimée de la vitesse V(t,z), respectivement $V_1(t,z)$ et $V_2(t,z)$.

**Claims**

1. A device for measuring and displaying physiological parameters of a blood flow in a vessel, comprising a first unit measuring for by ultrasonic echographyof speed V(t, z) of said blood flow versus time t and depth of examination z, the measurement of the speed V(t, z) being independent of the frequency of the ultrasonic wave used, and comprising a memory (304) for storing speed samples V(t, z), characterized in that it furthermore includes first circuits (403 and 409) for calculating the instantaneous flow rate Q(t) from said samples of the speed $V(t, z)$ and also a second unit for measuring the radial speeds $V_1(t, z)$ and $V_2(t, z)$ of shift of the two walls of the vessel which diametrically bound said blood flow, memories (504) for storing the values of the speeds $V_1(t, z)$ and $V_2(t, z)$, second calculating circuits comprising a circuit (604) for calculating the local energy $E'(t,z)$ of the amplified echo signal $S_n$ from which fixed echos have not been removed, which calculates: $E'(t,z) = \Sigma_n S^{2n}(t,z)$, followed by a circuit (605) for calculating the respective thicknesses $d_1 = z_4\text{-}z_3$ and $d_2 = z_6\text{-}z_5$ of said walls, constituted by a threshold detector for $E'_0$ which is adjustable for the determination of the values $z_3, z_4, z_5, z_6$, two circuits (606, 607) for calculating the respective mean speed speeds

$$\hat{V}_1(t_0) \text{ and } \hat{V}_2(t_0)$$

of said walls for each time value $t_0$, formed by an adder giving $\Sigma_{d1} V_1(t, z)$ and $\Sigma_{d2} V_2(t, z)$, respectively and by a divider which divides by $M_1$ and $M_2$, respectively $M_1$ and $M_2$ being the number of measuring samples of the segment $[z_3, z_4]$ and $[z_5, z_6]$, respectively a circuit (608, 609) for calculating the shift of each wall, formed by an adder producing

$$D_1(t) = \Sigma_t \hat{V}_1(t_0) \text{ and } D_2(t) = \Sigma_t \hat{V}_2(t_0),$$

and a circuit for calculating the symmetrical shift of the walls, formed by a subtractor (610) and a divide-by-2 divider (611) giving, in the form of time samples, the variation of the instantaneous radius of said vessel, $\Delta r(t) = (D_2(t)\text{-}D_1(t))/2$, and means (701, 702) for displaying curves $Q(t)$ and $\Delta r(t)$ as a function of time $t$.

2. A measuring and display device as claimed in Claim 1, characterized in that said first circuits for calculating the instantaneous flow rate $Q(t)$ comprise a circuit (405) for calculating the local energy from which fixed echos $E(t, z)$ have been removed, followed by a circuit for calculating the diameter of the flow $D(t) = z_2(t)\text{-}z_1(t)$ constituted by a threshold detector of value $E_0$, $z_2(t)$ and $z_1(t)$ being defined as:

$$E(t, z_1(t)) = E(t, z_2(t)) = E_0,$$

a circuit (406) for calculating the mean flow speed $\hat{V}(t)$, constituted by an adder producing:

$$\Sigma_D V(t, z)$$

and by a divide-by-M divider, M being the number of measuring samples of the segment $[z_1, z_2]$, and a circuit (407) for calculating the flow rate $Q(t)$ including, on the one hand, a circuit (408) for calculating the centre of gravity $z_0(t)$ of the flow which is formed by an adder giving $\Delta_D V(t, z)z$ and a divider dividing by the mean speed $\hat{V}(t)$, and on the other hand an adder (409) producing:

$$\Delta_D V(t, z)|Z\text{-}Z_0|$$

followed by a multiplier multiplying by a constant A which is a function of the angle $\theta$ between the flow and the ultrasonic beam.

3. A measuring and display device as claimed in Claim 1 or 2, characterized in that it furthermore includes means (702) for displaying the curve of the cardiac cycle CC, parametered versus time, formed by the points obtained by plotting on the ordinates the values of the function $\Delta r(t)$ in a linear relationship with the variation of the instantaneous pressure in the vessel and on the abscissae the values of the function of said instantaneous flow rate $Q(t)$.

4. A measuring and display device as claimed in Claim 3, characterized in that it furthermore includes a circuit (703) for calculating the cardiac efficiency EFF defined as:

$$EFF = 1\text{-}E_p/E_T$$

in which $E_p$ is equal to the surface $S_1$ of said cardiac cycle and $E_T$ is equal to the surface of the cycle increased by the adjacent surface $S_2$ subtended by the cycle in the direction of the axis of the ordinates.

5. A measuring and display device as claimed in any one of the Claims 1 to 4, characterized in that it also includes a device (704) for detecting the abscissae of a minimum, at the instant $t_1$, of a subsequent maximum, at the instant $t_3$, and of a second subsequent minimum, at the instant $t_2$, of the function $\Delta r(t)$, the duration $\Delta t = t_2\text{-}t_1$ being the duration of a cardiac cycle, a Fourier-series decomposition processor (705) which receives the time-sampled signals $\Delta r(t)$ and $Q(t)$ between the instants $t_1$ and $t_2$ and derives therefrom frequency samples $\Delta r(kf_0)$ and $Q(kf_0)$, $f_0 = 1/\Delta t$ being the fundamental frequency of the cardiac cycle and k the order of the harmonic under consideration, and an impedance calculating processor (708) which, for each harmonic, supplies the amplitude $\Delta r_k/Q_k$ and the phase $Arg(\Delta r_k/Q_k)$ of the impedance of the analysed vessel section.

6. A measuring and display device as claimed in Claim 5, characterized in that it furthermore includes means (709) for displaying the amplitude and the phase of the impedance point-by-point for each harmonic of the cardiac cycle.

7. A measuring and display device as claimed in any one of the Claims 1 to 6, characterized in that the first flow speed measuring unit and the second measuring unit for measuring the speed of shift of the walls comprise a respecting intercorrelation circuit (302, 502) supplying, on the basis of two consecutive echos, values of correlation functions, and a multiplexing-interpolating circuit (303, 503) supplying, on the basis of said correlation values, an estimate of the speed V(t, z), or $V_1(t, z)$ and $V_2(t, z)$, respectively.

## Patentansprüche

1. Vorrichtung zur Messung und Visualisierung von physiologischen Parametern einer Blutströmung in einem Gefäß, die für Ultraschallechographie eine erste Einheit zur Messung (300) der Geschwindigkeit V(t,z) der genannten Blutströmung als Funktion der Zeit t und der Untersuchungstiefe z umfaßt, wobei die Messung der Geschwindigkeit V(t,z) von der verwendeten Ultraschallwellenfrequenz unabhängig ist, und einen Speicher (304) zur Speicherung der Geschwindigkeitsabtastungen V(t,z), dadurch gekennzeichnet, daß sie außerdem in Kombination mit ersten Schaltungen (403 bis 409) zur Berechnung einer aus den genannten Geschwindigkeitsabtastungen hergeleiteten momentanen Durchflußmenge Q(t) sowie einer zweiten Einheit zur Messung (500) der radialen Geschwindigkeit $V_1(t,z)$ und $V_2(t,z)$ einer Ortsveränderung der beiden Gefäßwände, die die genannte Blutströmung diametral begrenzen, Speicher (504) zur Speicherung der Geschwindigkeitswerte $V_1(t,z)$ und $V_2(t,z)$ umfaßt, wobei zweite Berechnungsschaltungen eine Schaltung zur Berechnung (604) der lokalen Energie E'(t,z) des verstärkten, von Festechos freien Echossignals $S_n$ umfassen, die

$$E'(t,z)=\sum_n S_n^2(t,z)$$

berechnet, und auf die eine Schaltung zur Berechnung (605) der jeweiligen Dicken $d_1 = z_4 - z_3$ und $d_2 = z_6 - z_5$ der genannten Wände, die von einem Detektor eines für die Bestimmung der Werte $z_3$, $z_4$, $z_5$, $z_6$ einstellbaren Schwellenwertes E'$_0$ gebildet wird, folgt, zwei Schaltungen (606, 607), die von einem $\Sigma_{d1} V_1(t,z)$ (beziehungsweise $\Sigma_{d2} V_2(t,z)$) liefernden Addierer und einem durch $M_1$ beziehungsweise $M_2$ teilenden Dividierer gebildet werden, zur Berechnung der jeweiligen durchschnittlichen Geschwindigkeiten $\hat{V}_1(t_0)$ und $\hat{V}_2(t_0)$ der genannten Wände für jeden Zeitwert $t_0$, wobei $M_1$ und $M_2$ die Anzahl der Meßabtastungen in einem Segment $[z_3,t_4]$ beziehungsweise $[z_5, z_6]$ sind, eine Schaltung zur Berechnung (608, 609) der Ortsveränderung jeder Wand, gebildet von einem Addierer, der

$$D_1(t) = \sum_t \hat{V}_1(t_0) \text{ und } D_2 = \sum_t \hat{V}_2(t_0)$$

liefert, und eine Schaltung zur Berechnung der Ortsveränderung der symmetrischen Wände, die von einem Subtrahierer (610) und einem durch 2 teilenden Dividierer (611) gebildet wird und in Form von zeitlichen Abtastungen die augenblickliche Strahlveränderung des genannten Gefäßes $\Delta r(t) = (D_2(t)-D_1(t))/2$ liefert, und Mittel zur Visualisierung (701, 702) der Kurven Q(t) und $\Delta r(t)$ als Funktion der Zeit t.

2. Vorrichtung zur Messung und Visualisierung nach Anspruch 1, dadurch gekennzeichnet, daß die genannten ersten Schaltungen zur Berechnung der momentanen Durchflußmenge Q(t) eine Schaltung zur Berechnung (405) der von Festechos freien lokalen Energie E(t,z) umfassen, auf die eine Schaltung zur Berechnung des Durchmessers der Strömung $D(t) = z_2(t)-z_1(t)$ folgt, die von einem Detektor für einen Schwellenwert $E_0$ gebildet wird, wobei $z_2(t)$ und $z_1(t)$ durch

$$E(t,z_2(t)) = E(t,z_2(t)) = E_0$$

definiert sind, eine Schaltung zur Berechnung (406) der Durchschnittsgeschwindigkeit $\hat{V}(t)$ der Blutströmung, die von einem

$$\Sigma_D V(t,z)$$

liefernden Addierer und von einem durch M teilenden Dividierer gebildet wird, wobei M die Anzahl der Meßabtastungen im Segment $[z_1, z_2]$ ist, und eine Schaltung (407) zur Berechnung der Durchflußmenge Q(t), die erstens eine Schaltung zur Berechnung (408) des Schwerpunktes $z_0(t)$ der Strömung umfaßt, gebildet von einem $\Delta_D V(t,z)z$ liefernden Addierer und einem durch die Durchschnittsgeschwindigkeit $\hat{V}(t)$ teilenden Dividierer, und zweitens einen $\Delta_D V(t,z)|z-z_0|$ liefernden Addierer (409), auf den ein Multiplizierer folgt, der mit einer Konstante A abhängig vom Winkel $\theta$ zwischen der Blutströmung und dem Ultraschallwellenbündel multipliziert.

3. Vorrichtung zur Messung und Visualisierung nach Anspruch 1 oder 2, dadurch gekennzeichnet, daß sie außerdem Mittel zu Visualisierung (702) der Kurve des Herzzyklus umfaßt, in zeitlicher Parameterdarstellung, bestehend aus den Punkten, die erhalten werden, indem die Werte der Funktion $\Delta r(t)$ in linearer Beziehung zur momentanen Druckveränderung im Gefäß als Ordinaten und die Werte der Funktion der genannten momentanen Durchflußmenge Q(t) als Abszissen eingetragen werden.

4. Vorrichtung zur Messung und Visualisierung nach Anspruch 3, dadurch gekennzeichnet, daß sie außerdem eine Schaltung (703) zur Messung der durch

$$EFF = 1 - E_p/E_T$$

definierten Herzeffizienz EFF umfaßt, wobei in dieser Gleichung $E_p$ gleich der Fläche $S_1$ des genannten Herzzyklus und $E_T$ gleich der auf die angrenzende Fläche $S_2$ ausgedehnte, durch den Zyklus in Richtung der Achsen der Ordinaten überspannte Fläche des Zyklus ist.

5. Vorrichtung zur Messung und Visualisierung nach einem der Ansprüche 1 bis 4, <u>dadurch gekennzeichnet</u>, daß sie außerdem eine Detektionsvorrichtung (704) für die Abszissen eines Minimums zu einem Zeitpunkt $t_1$, eines folgenden Maximums zu einem Zeitpunkt $t_2$ und eines folgenden zweiten Minimums zum Zeitpunkt $t_3$ der Funktion $\Delta r(t)$ umfaßt, wobei die Dauer $\Delta t = t_2 - t_1$ die eines Herzzyklus ist, einen seriellen Fourier-Zelegungs-Prozessor (705), der die in der Zeit $\Delta r(t)$ und $Q(t)$ zwischen $t_1$ und $t_2$ abgetasteten Signale empfängt und daraus die Frequenzabtastung $\Delta r(kf_0)$ und $Q(kf_0)$ ermittelt, wobei $f_0$ die Grundfrequenz des Herzzyklus und $k$ der Rang der betrachteten Harmonischen ist, und einen Prozessor (708) zur Impedanzberechnung, der für jede Harmonische die Amplitude $\|\Delta r_k/Q_k\|$ und die Phase $Arg(\Delta r_k/Q_k)$ der Impedanz des untersuchten Gefäßbereiches liefert.

6. Vorrichtung zur Messung und Visualisierung nach Anspruch 5, <u>dadurch gekennzeichnet</u>, daß sie außerdem Mittel (709) zur punktweisen Visualisierung der Amplitude und der Phase der Impedanz für jede Harmonische des Herzzyklus umfaßt.

7. Vorrichtung zur Messung und Visualisierung nach einem der Ansprüche 1 bis 6, <u>dadurch gekennzeichnet</u>, daß die erste Einheit zur Messung der Strömungsgeschwindigkeit und die zweite Einheit zur Messung der Ortsveränderungsgeschwindigkeit der Wände beide eine Korrelationsschaltung (302, 502) umfassen, die aufgrund von zwei aufeinanderfolgenden Echos Korrelationsfunktionswerte liefert, und eine Multiplex-Interpolationsschaltung (303, 503), die aufgrund der genannten Korrelationswerte eine Schätzung der Geschwindigkeit $V(t,z)$ beziehungsweise $V_1(t,z)$ und $V_2(t,z)$ liefert.

FIG.1a

FIG.1b

FIG.4a

FIG.4b

FIG.2

FIG.3